Europäisches Patentamt

European Patent Office

Office européen des brevets

(19)

(11) Numéro de publication: **0 318 358**
**A1**

## (12) DEMANDE DE BREVET EUROPEEN

(21) Numéro de dépôt: **88402877.0**

(22) Date de dépôt: **16.11.88**

(51) Int. Cl.4: **A 61 M 1/00**

(30) Priorité: **23.11.87 FR 8716195**

(43) Date de publication de la demande:
**31.05.89 Bulletin 89/22**

(84) Etats contractants désignés:
**AT BE CH DE ES FR GB GR IT LI LU NL SE**

(71) Demandeur: **Société dite : LG MEDICAL S.A.**
**Avenue des Temps Modernes B.P. 31**
**F-86361 Chasseneuil Cédex (FR)**

(72) Inventeur: **Metais, Joel**
**La Cottencière Berthegon**
**F-86420 Monts Sur Guesnes (FR)**

**Lehmann, Gérard**
**6, route de Poitiers**
**F-86170 Avanton (FR)**

(74) Mandataire: **Lerner, François et al**
**5, rue Jules Lefèbvre**
**F-75009 Paris (FR)**

(54) Dispositif implantable pour accès au système circulatoire sanguin et ses différents éléments pour son utilisation.

(57) L'invention concerne un dispositif implantable pour accès au système circulatoire sanguin.

L'invention comprend un corps implantable (1) en forme générale de té (T) comportant un puits (3), séparé par une paroi (8) de fond de la branche (2) du té reliée aux prothèses vasculaires (11, 12). La connexion/déconnexion du dispositif peut être obtenue au moyen d'un tournant (15) qui peut être actionné à partir du puits et éventuellement changé sans perte de sang.

L'invention s'applique notamment aux traitements de dialyse.

FIG. 3

EP 0 318 358 A1

## Description

## DISPOSITIF IMPLANTABLE POUR ACCES AU SYSTEME CIRCULATOIRE SANGUIN ET SES DIFFERENTS ELEMENTS POUR SON UTILISATION

L'invention a pour objet un dispositif implantable pour accès au système circulatoire sanguin du type comprenant essentiellement un raccord en té (T) dont les extrémités débouchantes de la barre ou branche sont reliées à des veines, artères ou prothèses vasculaires et dont l'extrémité de la hampe, formant puits d'accès, peut être connectée à tout système extérieur d'échange, par exemple pour la dialyse du sang d'un patient et dans lequel la connexion/déconnexion avec l'extérieur est obtenue au moyen d'un organe monté dans ledit puits formant tournant comportant deux orifices qui, selon la position en rotation du tournant sont mis en communication ou hors de communication avec le volume intérieur de la branche du té et qui, d'autre part, peuvent être réliés à l'entrée et à la sortie dudit système extérieur.

De tels dispositifs sont connus et par exemple décrits au brevet US-A-4 108 173. Ces dispositifs présentent pour le patient de grands avantages d'utilisation et de confort, évitant à chaque intervention sur le système circulatoire l'obligation de trouver un endroit possible où effectuer une nouvelle intervention avec, en particulier pour le cas de la dialyse, des aiguilles de diamètre particulièrement important. Mais les dispositifs connus sont de conception très complexe, sont par suite extrêmement coûteux et présentent diverses difficultés d'utilisation, notamment lors d'une révision des pièces qui les composent ou d'un remplacement nécessaire de tel ou tel organe sujet à usure.

Le dispositif conforme à l'invention se caractérise par rapport aux dispositifs du type général ci-dessus décrits en ce que le tournant se présente sous la forme d'un disque sensiblement cylindrique circulaire dont le diamètre extérieur correspond sensiblement au diamètre intérieur du puits comportant des moyens coopérant avec des moyens complémentaires dudit puits pour assurer au moins, dans la position de déconnexion du dispositif un appui en pression du disque sur le fond du puits et une étanchéité de la fermeture du puits. Avantageusement et selon une réalisation simple à mettre en oeuvre, les moyens complémentaires en question consistent en des rampes formées sur le disque coopérant avec des ergots formés sur la paroi intérieure du puits.

Selon une caractéristique essentielle de l'invention, le dispositif comprend en outre un dispositif connecteur pour la liaison au système extérieur comprenant un bouchon pouvant venir s'engager de façon étanche dans le puits, lequel bouchon est traversé de façon étanche par deux broches creuses susceptibles de passer à travers les orifices précités formés dans le tournant; ce connecteur comprend en outre des moyens de guidage et d'orientation dans le puits tels qu'au moins une rainure formée sur sa paroi latérale coopérant avec un doigt prévu sur la paroi du puits imposant un mouvement d'introduction du connecteur comportant au moins un premier déplacement d'enfoncement jusqu'à pénétration des broches dans les orifices du tournant alors en position d'obturation, puis un mouvement de rotation jusqu'à amener le tournant en position d'ouverture, puis un nouvel enfoncement rectiligne jusqu'à pénétration des broches au niveau souhaitable dans le volume intérieur de la branche du té, les mouvements de retrait du connecteur étant inverses de ceux de l'introduction et entraînant simultanément l'obligation de positionner en obturation le tournant avant retrait du connecteur.

Il apparaît que de la sorte l'intervention, c'està-dire l'opération de connexion et de déconnexion avec le système extérieur par exemple de dialyse ou de perfusion s'effectue très simplement, sans possibilité d'erreur de manoeuvre ni difficulté particulière.

Selon une caractéristique très avantageuse de l'invention, le dispositif comprend en outre un dispositif extracteur comportant comme le connecteur un bouchon pouvant venir s'engager de façon étanche dans le puits, lequel bouchon comporte deux orifices pour le passage de broches susceptibles de passer à travers les orifices précités formés dans le tournant ; cet extracteur comprend en outre des moyens de guidage et d'orientation dans le puits tels qu'au moins une rainure formée sur sa paroi latérale coopérant avec un doigt prévu sur la paroi du puits imposant un mouvement d'introduction de l'extracteur comportant au moins un premier déplacement d'enfoncement jusqu'à pénétration des broches dans les orifices du tournant alors en position d'obturation, puis un mouvement de rotation jusqu'à amener le tournant en position d'ouverture ; dans cette position, les broches peuvent être enfoncées à travers les orifices de la paroi de fond du puits qu'elles obturent puis, les broches restant en place, l'extracteur peut être retiré dans un mouvement d'extraction parallèle à l'axe du puits en entraînant avec lui le tournant, grâce à la prévision d'au moins une rainure parallèle à l'axe du puits et dans laquelle est engagé ledit doigt dans cette position en rotation de l'extracteur. On comprend que de cette façon, il est possible de procéder de manière simple au changement du tournant, lequel constitue la seule pièce d'usure du système, et ceci sans perte de sang.

L'invention et sa mise en oeuvre apparaîtront plus clairement à l'aide de la description qui va suivre faite à titre d'illustration d'exemples de réalisation.

Dans ces dessins :
- la figure 1 montre de façon schématique et en perspective le raccord en té connecté à deux prothèses vasculaires ;
- la figure 2 montre à plus grande échelle, en vue extérieure et perspective, le seul raccord en té ;
- la figure 3 montre en coupe, dans le plan médian du té contenant l'axe du puits et l'axe de la branche, le té connecté aux prothèses vasculaires et dans le puits duquel est placé en position d'obturation le tournant ;

- la figure 4 montre, à plus grande échelle, vu de côté, le tournant ;

- la figure 5 montre le même tournant en vue perspective ;

- la figure 6 montre en vue perspective l'ensemble du connecteur avec sa bague de verrouillage ;

- la figure 7 montre à plus grande échelle et sous un autre angle de vue avec arrachements partiels le même connecteur ;

- la figure 8 montre en vue en coupe longitudinale le connecteur avec sa bague de verrouillage ;

- les figures 9, 10, 11 et 12 sont des schémas expliquant la mise en place et l'introduction du connecteur sur le dispositif ;

- la figure 13 est une vue en perspective montrant un extracteur utilisé selon l'invention ;

- les figures 14 à 18 sont des schémas expliquant l'utilisation de l'extracteur ;

- la figure 19 est en vue en coupe montrant le dispositif de l'invention avec son bouchon de blocage en place ;

- les figures 20, 21, 22 montrent de façon schématique et en vue par dessus un tournant selon une variante de réalisation occupant trois positions de rotation différentes dans le puits ;

- la figure 23 montre en vue perspective le tournant modifié des figures 20 à 22 ;

- la figure 24 montre un détail selon la flèche XXIV de la figure 23 ;

- la figure 25 est une coupe à plus grande échelle faite dans le plan XXV-XXV de la figure 23 ;

la figure 26 montre vu de bout l'extracteur modifié utilisable avec le tournant des figures 23 à 25.

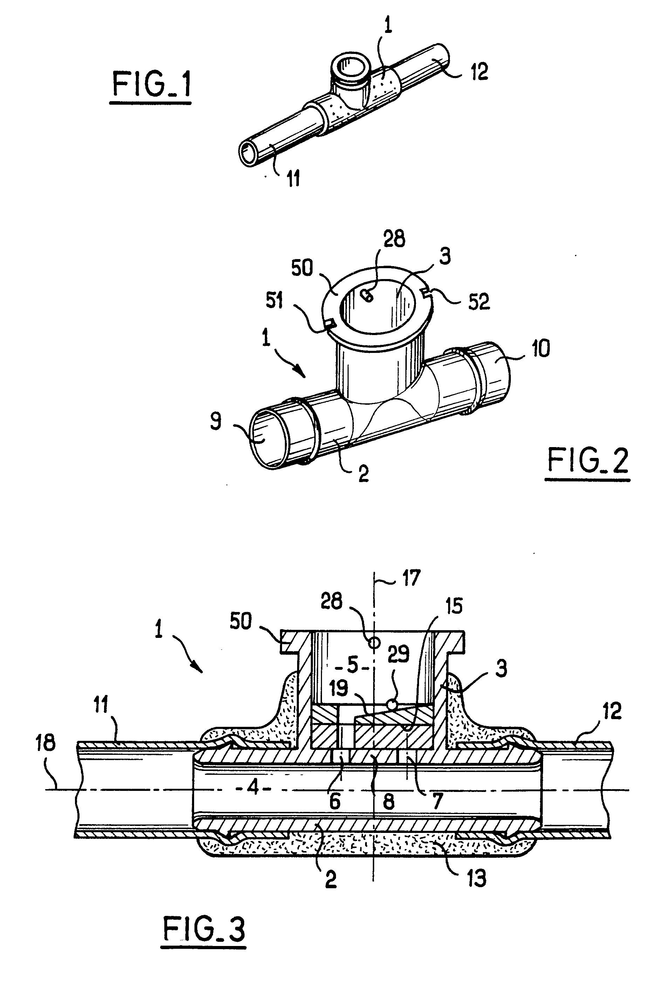

On se reportera tout d'abord aux figures 1 à 3 des dessins.

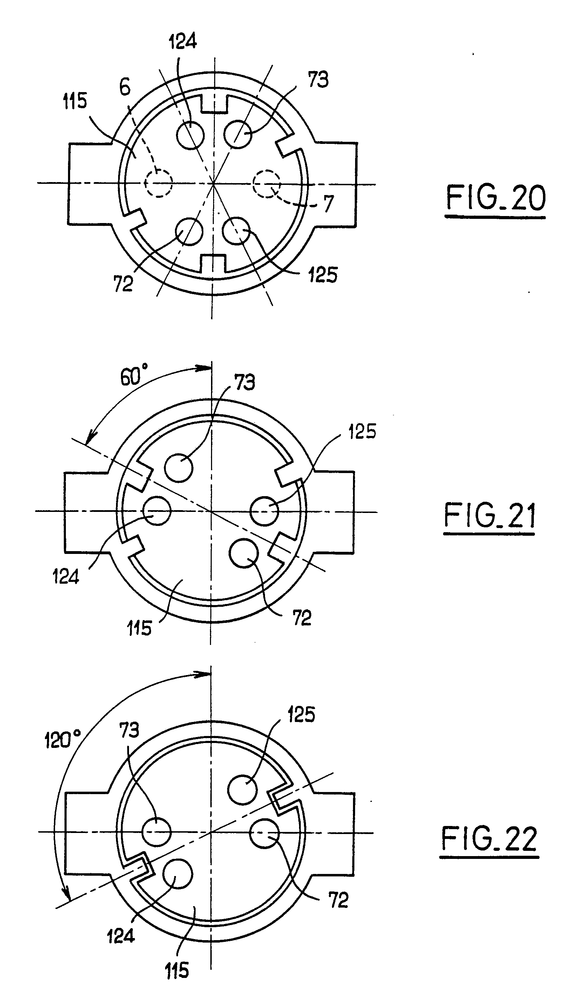

Le dispositif implantable pour accès au système circulatoire sanguin d'un patient comprend essentiellement un corps en té 1 comprenant essentiellement une branche 2 formant la barre du té communiquant avec un puits 3 formant la hampe du té. Comme on le voit plus clairement à la figure 3, la communication entre le volume intérieur 4 de la branche 2 et le volume intérieur 5 du puits 3 se fait au moyen de deux orifices 6, 7, formés à travers une paroi 8 de séparation des volumes 4 et 5. A ses extrémités 9 et 10, la branche 2 est d'autre part connectée de préférence à des prothèses vasculaires 11, 12, préalablement ménagées comme il est connu. Un revêtement compatible, par exemple de velours de Dacron (marque déposée) ou autre matériau biocompatible, recouvre la partie implantée sous la peau du dispositif, comme repéré en 13. On peut également se référer à la figure 19, montrant le dispositif 1 en place implanté sous la peau référencée 14 d'un patient.

En revenant à la figure 3, on aperçoit également à cette figure le tournant 15 en position de blocage dans laquelle les passages 6 et 7 sont obturés par ce tournant.

En faisant référence aux figures 3, 4 et 5, on comprend plus facilement la fonction et l'action du tournant 15.

Selon l'exemple de réalisation illustré, le tournant 15 est de façon générale symétrique autour de son axe vertical 16. Dans sa position d'introduction dans le puits 3, l'axe 16 du tournant 15 correspond à l'axe 17 du puits, lequel est perpendiculaire à l'axe 18 de la branche 2.

Sur sa face supérieure le tournant 15 comporte deux rampes 19, 20 débutant chacune dans une saignée 21, 22, parallèles à l'axe 16 et venant se terminer de niveau avec la face extérieure libre (supérieure comme représentée aux dessins) 23 du tournant.

Dans le tournant, sont également ménagés deux orifices 24, 25, sensiblement symétriques et qui traversent tout le tournant. On notera tout de suite, mais on y reviendra plus loin, que dans la position d'utilisation ou connexion du dispositif, les orifices 24, 25, du tournant viennent en face et communiquent avec les orifices 6, 7 de la paroi 8 du fond du puits, ces orifices 6, 7, 24, 25, étant de préférence cylindriques circulaires de même diamètre.

Dans l'exemple de réalisation illustré, le tournant 15 est composé en deux parties, à savoir une plaque d'appui 15a et un joint 15b. La plaque d'appui peut être constituée en différents matériaux tels notamment que titane ou une matière plastique dure de qualité appropriée tel que polyéthylène ou polypropylène par exemple. La rondelle 15b peut être une rondelle de silicone qui peut être collée ou surmoulée sur la pièce 15a. Dans ce cas la pièce ainsi constituée présente dans son ensemble une certaine élasticité permettant une bonne étanchéité par compression lors de la fermeture du tournant comme il sera expliqué plus loin. Mais dans d'autres réalisations, les pièces 15a et 15b peuvent être constituées en un seul bloc d'une même matière, par exemple tel que graphite, céramique, polytétrafluoréthylène (PTFE), etc... Cette pièce monobloc est alors rigide et l'étanchéité à la fermeture est simplement obtenue du fait de la parfaite adaptation l'une sur l'autre de la face inférieure polie du tournant et de la face en regard formant siège du fond du puits.

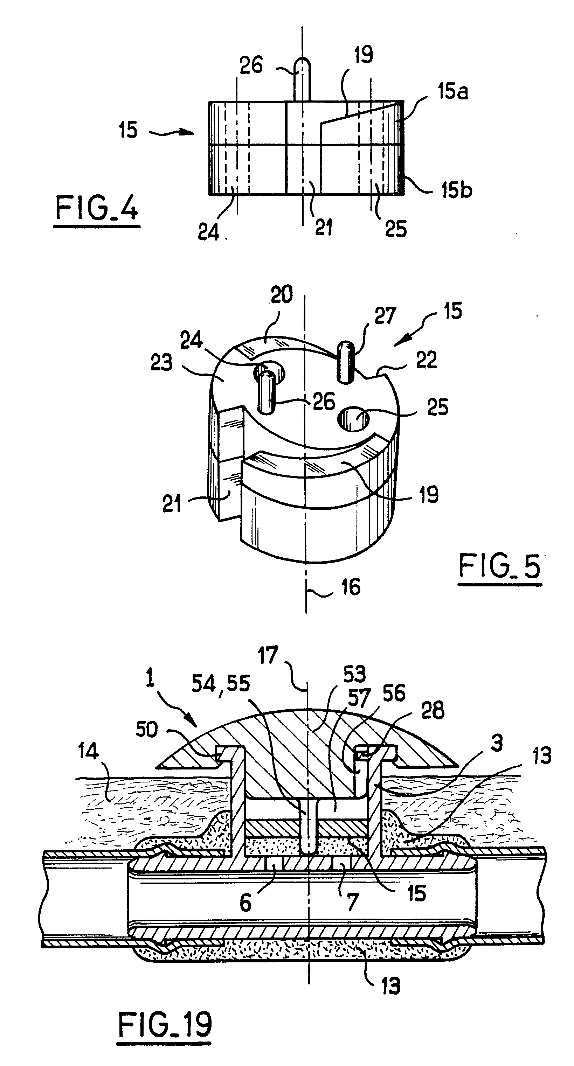

Enfin en retournant aux figures 4 et 5, on aperçoit sur le dessus du tournant faisant saillie au-dessus de la face supérieure 23, deux pions 26, 27, sensiblement cylindriques et dont le rôle apparaîtra plus loin.

En revenant à la figure 3, on aperçoit également faisant saillie sur la paroi intérieure du puits 3 à l'intérieur du volume 5, respectivement un doigt 28 servant au guidage et à la mise en place du connecteur et de l'extracteur comme il apparaîtra plus loin et l'un 29 de deux ergots disposés en symétrie sur la paroi du puits et venant coopérer avec les rampes 19, 20 du tournant comme il sera décrit plus loin.

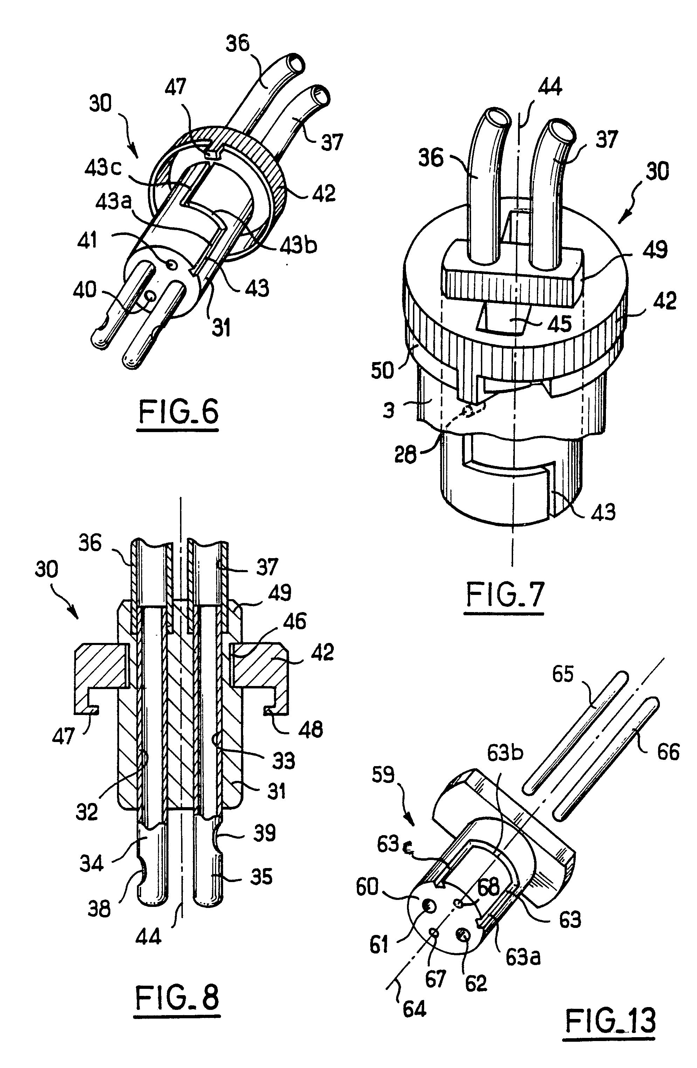

En faisant référence aux figures 6 à 8, on décrira maintenant le dispositif formant connecteur et partie du dispositif complet conforme à l'invention. Le connecteur repéré dans son ensemble 30 comprend essentiellement un bouchon 31 pouvant venir s'engager de préférence de façon sensiblement étanche dans le puits 3. Il peut être constitué par exemple en une matière telle que du polyéthylène, la matière formant le puits 3 et la branche 2 du corps en té pouvant être constituée par exemple par du titane à revêtement pyrolite ou encore du graphite à revêtement pyrolite, ou encore du polytétrafluoréthylène (PTFE). A l'intérieur du bouchon 31 du connecteur sont formés deux orifices 32, 33, permettant le passage étanche de deux broches 34, 35, par exemple en acier inoxydable de qualité appropriée et qui seront reliées aux tubes 36, 37 de connexion avec l'extérieur, par exemple un appareil de dialyse (non représenté).

Le dimensionnement et le positionnement relatifs des broches 34 et 35 correspondent évidemment aux dimensionnement et positionnement relatifs des orifices 24, 25, du tournant dans lesquels ces broches pourront pénétrer et qu'elles pourront traverser de façon sensiblement étanche.

A l'extrémité inférieure des broches 34, 35, on aperçoit les orifices légèrement décalés en hauteur 38, 39, pour l'entrée et la reprise par exemple du sang dialysé lors de l'utilisation du dispositif.

Sur sa face inférieure, comme on le voit plus clairement à la figure 6, le bouchon 31 du connecteur comprend deux trous borgnes 40, 41, pour le logement avec un jeu suffisant des pions 26, 27 du tournant. A la partie supérieure du connecteur, on aperçoit la bague de verrouillage 42, permettant le blocage avantageusement en étanchéité du connecteur sur le site lors d'une opération par exemple de dialyse.

Enfin, en se référant plus particulièrement à la figure 6, on aperçoit sur la paroi latérale du bouchon 31 du connecteur une rainure formée en creux 43 comportant successivement trois segments 43a, 43b et 43c dessinant une marche d'escalier, le premier segment 43a étant dirigé parallèlement à l'axe 44 longitudinal du connecteur ainsi que le dernier segment 43c, le segment médian 43b contenu dans un plan perpendiculaire à l'axe 44 dessinant un quart de tour sur la paroi extérieure du bouchon 31.

Aux figures 7 et 8, on aperçoit également le montage de la bague de blocage 42 sur le bouchon 31 permettant la rotation dans une cannelure 46 formée vers la partie supérieure du bouchon 31, ainsi que la prévision d'une encoche 45 dans la bague 42 permettant dans une position relative de rotation de la bague 42 par rapport au bouchon 31 la mise en place de la bague 42 dans sa cannelure 44 de guidage, et ce grâce à la forme conjuguée de l'épaulement supérieur 49 du bouchon 31. Aux figures 6 à 8, on aperçoit également deux doigts 47, 48, formant retours pouvant venir s'engager après mise en place du connecteur dans le puits 3 sous la collerette 50 du puits.

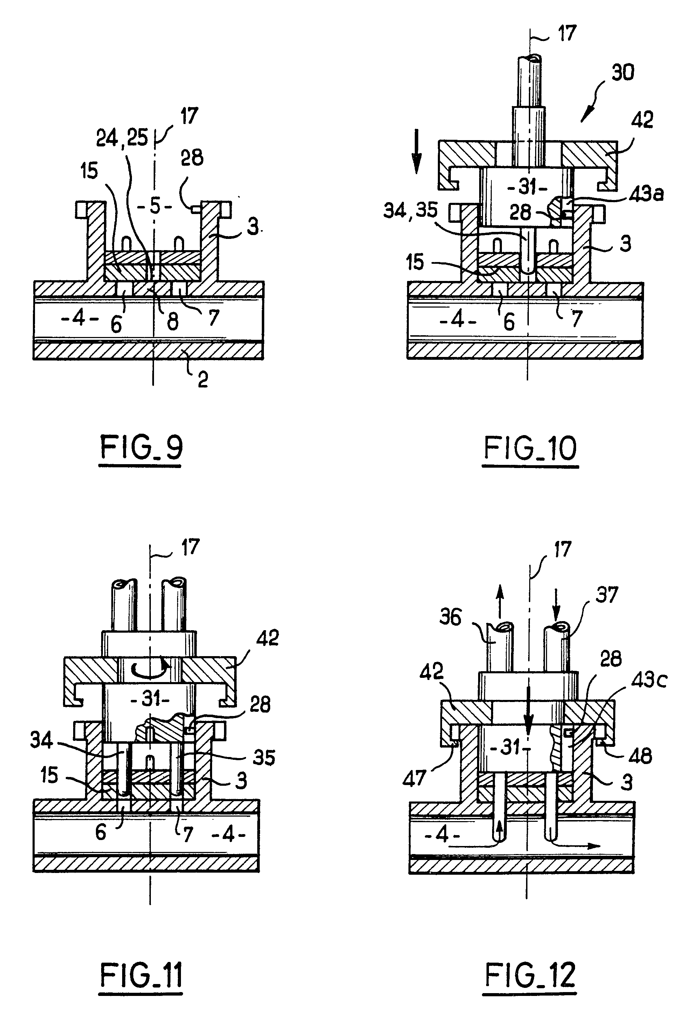

En faisant référence maintenant, plus particulièrement aux figures 9 à 12, on décrira le fonctionnement du dispositif.

En se reportant tout d'abord à la figure 9, on a supposé le dispositif en place, le tournant 15 étant en position d'obturation. Dans cette position les orifices 24, 25 du tournant qui sont visibles l'un derrière l'autre dans l'axe 17 du puits, ne communiquent pas avec les orifices 6, 7 de la paroi 8 du fond du puits. Les volumes 5 et 4 du puits et de la branche du té ne sont donc pas en liaison. Au contraire, ils sont isolés de façon étanche, grâce à l'application du tournant en pression sur le fond du puits. Ceci est obtenu comme il apparaît plus clairement par comparaison des figures 3 et 5, du fait que dans cette position en rotation du tournant 15, les ergots 29 sont engagés sous les rampes 19, 20 du tournant dans leur partie haute venant donc presser fermement par cet effet de rampe le fond plat du tournant 15 contre le fond plat en regard du puits.

En se reportant maintenant à la figure 10, on a illustré la phase dans laquelle on vient présenter le connecteur 30 par dessus le puits. De façon plus précise on introduit le bouchon 31 du connecteur dans la paroi du puits. Mais cette introduction ne peut se faire que dans la position angulaire pour laquelle la rainure 43 (figure 6) et plus précisément le segment 43a de cette rainure vient en regard du doigt 28 de guidage du connecteur. Cette position correspond très précisément à la position pour laquelle les branches 34, 35 (visibles l'une derrière l'autre dans l'axe 17 du puits) viennent en alignement avec les orifices 24, 25 du tournant, dans lesquels orifices lesdites broches peuvent s'engager. La longueur du segment 43a est calculée de façon que lorsque le doigt 28 vient au fond de cette rainure , pénétrant à l'intérieur du segment 43b, les broches 34, 35 sont venues sensiblement traverser toute l'épaisseur du tournant 15.

A ce moment, on ne peut alors poursuivre l'enfoncement des broches et du bouchon 31 dans le puits 3, que si l'on entraîne préalablement en rotation sur un quart de tour comme indiqué à la figure 11 le connecteur. Dans un tel mouvement, l'ergot 28 décrit le segment 43b de la rainure 43. Et simultanément, les broches 34, 35, qui ont pénétré dans les orifices 24, 25, du tournant impriment à celui-ci un quart de tour. Cette rotation a pour effet :
- de dégager l'action de la pression des ergots 29 sur les rampes 19, 20,
- d'amener en regard les orifices 24, 25, du tournant avec les orifices 6, 7, de communication du fond du puits.

Il est à noter cependant, que les orifices 24, 25 sont actuellement en fait obturés de façon étanche par les broches 34, 35.

Dans cette position, il devient alors possible, comme illustré à la figure 12, d'enfoncer davantage le bouchon 31 dans le puits 3, le doigt 28 étant venu en effet en regard du segment 43c de la rainure 43. Dans un tel mouvement d'enfoncement, les broches traversent alors les orifices 6, 7, du fond du puits et pénètrent dans le volume 4 réalisant la connexion du système extérieur avec les prothèses vasculaires 11, 12 (voir figure 3) en liaison avec le volume 4. Le système de dialyse peut alors fonctionner, les tubes 36 et 37 étant en liaison avec les prothèses vasculaires. Dans cette position, on abaisse alors la bague de verrouillage 42 sous la collerette 50 du puits, en faisant pénétrer les doigts de verrouillage en retour 47, 48 à travers les encoches 51, 52, ménagées à cet effet dans la collerette (voir figure 2) puis en faisant ensuite tourner la bague sous la collerette. Le connecteur est ainsi verrouillé en place sur le site.

Il est clair que les opérations de déconnexion se font de façon strictement inverse à celles de la connexion.

En bref, on déverrouillera d'abord la bague 42, on pourra alors retirer partiellement le connecteur, jusqu'à blocage du doigt 28 au fond du segment rectiligne 43c de la rainure 43 ; après quoi on effectue un quart de tour en sens inverse de celui décrit à la figure 11, le doigt 28 décrivant en sens inverse le segment 43b de la rainure 43 et les broches 34, 35, entraînant en même temps le tournant 15 en verrouillage positif dans le puits, du fait de l'engagement des rampes 19, 20, du tournant sous les ergots 29 de verrouillage. Lorsque le quart de tour complet est effectué, on peut alors retirer le connecteur, le segment 43a de rainure rectiligne étant à ce moment en face du doigt 28.

En se reportant à la figure 19, on voit qu'à ce moment, il est possible de verrouiller le dispositif dans cet état, en venant simplement presser par dessus la collerette 50 du puits 3 un bouchon 53 constitué en matière plastique de qualité appropriée, par exemple du polyéthylène injecté. Ce bouchon 53 comporte deux broches 54, 55, apparaissant dans la figure 19 l'une derrière l'autre dans l'axe 17 du puits 3. Ces broches ont un positionnement qui correspond à celui des orifices 24, 25 du tournant 15 dans cette position de fermeture. Le bon positionnement du bouchon 53 est assuré par une rainure 56 qui doit être engagée par dessus le doigt 28 de positionnement du connecteur comme il a été décrit ci-avant. De cette façon, on se prémunit contre tout déverrouillage intempestif du tournant 15. On notera, que dans espace compris sous le bouchon 53 et par dessus le tournant 15, espace repéré 57, on pourra introduire un désinfectant approprié.

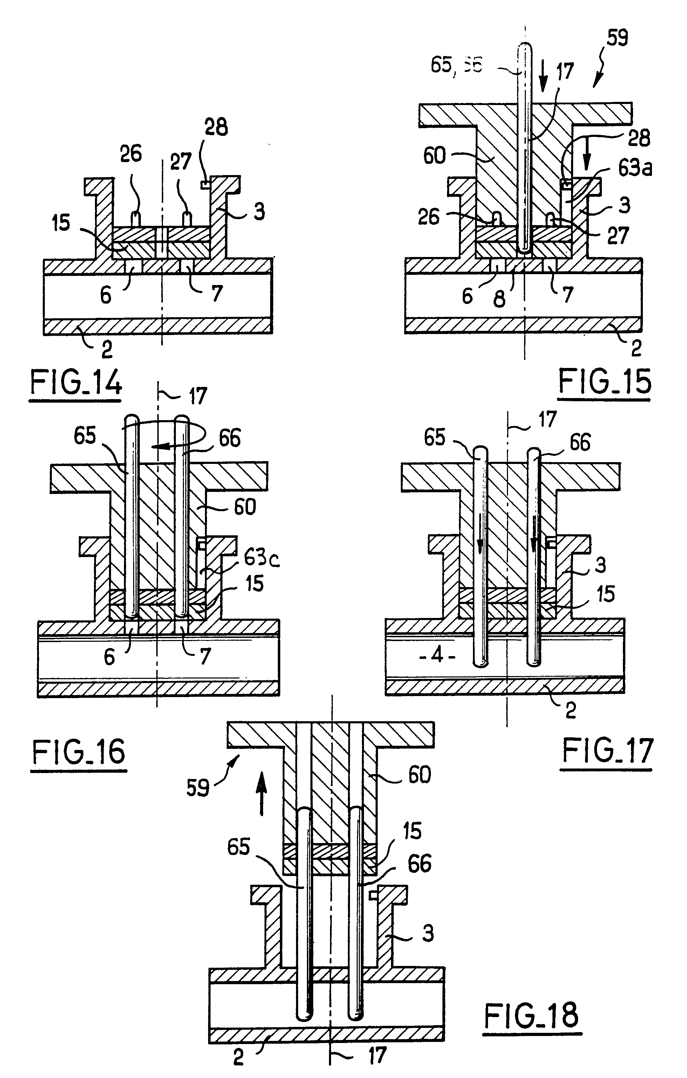

En faisant référence maintenant aux figures 13 à 18 on va décrire un dispositif complémentaire formant extracteur ainsi que son fonctionnement permettant en cas de besoin pour la maintenance du dispositif le changement du tournant 15.

En se référant à la figure 13, l'extracteur repéré dans son ensemble 59 se compose essentiellement d'un bouchon 60 percé de deux orifices 61, 62. Le bouchon 60 peut avoir des dimensions tout à fait semblables à celles du bouchon 31 du connecteur, et les orifices 61, 62 sont également avantageusement semblables en dimensions et positionnement aux orifices 32, 33 du connecteur.

Sur la paroi latérale de l'extracteur, on aperçoit une rainure 63 ayant une forme générale de U avec un premier segment 63a partant de la base du bouchon 60 et s'étendant sur une certaine longueur parallèlement à l'axe 64 du bouchon, un segment curviligne en quart de cercle 63b, situé dans un plan perpendiculaire à l'axe 64, puis un troisième segment 63c parallèle au segment 63a et revenant jusqu'à la base du bouchon 60.

A la figure 13 on aperçoit également deux broches, qui peuvent être de simples tiges pleines en acier 65, 66, susceptibles de s'engager en obturant sensiblement les orifices 61, 62, du bouchon. Ces broches 65, 66, ont sensiblement le même diamètre extérieur que les broches 34, 35, précédemment décrites en relation avec le connecteur 30.

A la figure 13 on aperçoit également décalés de 90° par rapport aux orifices 61, 62, deux trous borgnes 67, 68, dont on expliquera ci-après l'intérêt.

En se référant aux figures 14 à 18, on va décrire le fonctionnement de l'extracteur.

A la figure 14, on a représenté, comme à la figure 9, le dispositif, bouchon de protection 53 enlevé avec le tournant 15 en position d'obturation.

On vient alors positionner comme illustré à la figure 15, dans le puits, l'extracteur 59 en enfonçant le bouchon 60 de façon étanche dans le puits 3. Cette opération est effectuée en présentant l'ergot 28 dans la rainure 63a. Dans cette position angulaire relative, les broches 65, 66, qui ont été enfoncées à travers le bouchon 60 sont alignées l'une derrière l'autre dans l'axe 17 du puits et viennent également en alignement avec les orifices 24, 25 du tournant 15 dans la position d'obturation qu'il occupe. Elles peuvent être enfoncées jusqu'à venir porter sensiblement contre la paroi 8 de fond du puits. Quant aux pions 26, 27 faisant saillie sur la face supérieure 23 du tournant 15, ceux-ci viennent s'engager dans les trous borgnes 67, 68, précédemment mentionnés du bouchon 60. Avantageusement, les pions ont une forme sensiblement cylindrique circulaire, tandis que les trous borgnes 67, 68, de diamètre sensiblement équivalent sont légèrement tronconiques avec une section allant en se rétrécissant depuis le débouché du trou jusqu'au fond. De cette manière, lorsqu'on applique l'extracteur par dessus le tournant, les pions 26, 27 viennent se verrouiller élastiquement dans les trous borgnes 67, 68.

Comme illustré à la figure 16, on imprime alors un quart de tour au bouchon 60, les branches 65, 66 tournant simultanément en entraînant le tournant 15 dont les orifices viennent en regard des orifices 6, 7, formés dans le fond du puits.

Comme illustré à la figure 17, on enfonce alors les broches 65, 66, davantage, les faisant pénétrer à travers les orifices 6 et 7, qu'elles obturent venant séparer, de façon étanche le volume 4 de la branche par rapport à l'extérieur.

Il ne reste plus alors, comme illustré à la figure 18, qu'à retirer l'extracteur 59 en le soulevant parallèlement à l'axe 17 du puits, en le faisant glisser sur les broches 65, 66 qui restent en place. Dans ce mouvement, le tournant 15, verrouillé par les pions 26, 27, coincés dans les trous 67, 68, tronconiques de l'extracteur reste collé à ce dernier.

La mise en place d'un nouveau tournant de remplacement se fait en opérant de la façon exactement inverse de celle qui vient d'être décrite en relation aux figures 14 à 18, pouvant se résumer comme suit :
- mise en place sur un extracteur d'un nouveau tournant 15,
- introduction de l'extracteur et du tournant par dessus les broches 65, 66,
- retrait partiel des broches de façon qu'elles dégagent les orifices 6 et 7,

- rotation de 90° de l'extracteur avec fermeture simultanée du tournant au fond du puits,
- retrait de l'extracteur.

De la description qui précède, il apparaît que le dispositif de l'invention est de réalisation et d'utilisation particulièrement simples.

Selon la variante de réalisation illustrée aux figures 20 à 26, le tournant 115 présente quelques modifications par rapport au tournant 15 utilisé et illustré dans les figures précédentes.

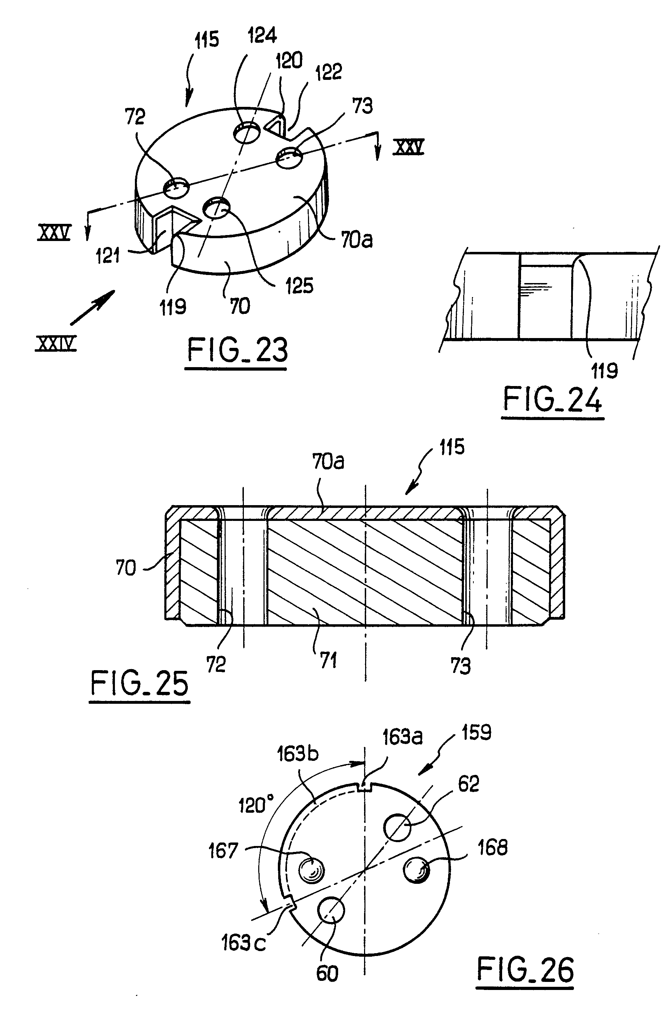

Il est constitué essentiellement d'une coupelle métallique, avantageusement en titane 70 dans laquelle est logée et maintenue, par exemple par légère compression, sertissage ou surmoulage une rondelle 71 en matière élastomère biocompatible telle qu'un silicone, en matière plastique convenable etc... La rondelle 71 dépasse légèrement en saillie en dessous de la jupe de la coupelle 70 comme il apparaît plus clairement à la figure 25.

Le tournant comporte deux orifices 124, 125 qui correspondent aux orifices 24, 25, de la réalisation précédemment décrite, ainsi que deux autres orifices 72, 73 de diamètre sensiblement équivalent et dont l'utilité va apparaître ci-après.

Comme il apparaît plus clairement à la figure 24 les deux saignées 121, 122 du tournant équivalentes aux saignées 21, 22 de la réalisation précédemment décrite présentent d'un côté un arrondi 119, 120 dont la fonction est sensiblement équivalente à celle des rampes 19, 20 de la réalisation précédente et comme il apparaîtra de la description qui va suivre du fonctionnement.

En se référant à la figure 25 on aperçoit également en face des orifices tels que 72, 73 du tournant qu'un certain arrondi et élargissement ont été ménagés dans la paroi 70a supérieure de la coupelle 70 pour faciliter l'introduction des broches à travers le tournant.

L'utilisation du dispositif ainsi modifié va être brièvement décrite.

En se référant à la figure 20 on a illustré la position dans laquelle on va pouvoir mettre en place le connecteur pour effectuer la dialyse comme il a été décrit précédemment notamment en référence aux figures 10 à 12.

On opère de façon tout à fait semblable à celle décrite dans le mode de réalisation précédent en présentant le connecteur dans la bonne position, en introduisant ensuite les deux broches 34, 35 du connecteur dans les orifices correspondants 124, 125 du tournant puis en imprimant ensuite au système une rotation de 60° comme indiqué à la figure 21 (rotation qui était de 45° dans le mode de réalisation décrit précédemment). Après rotation de 60° les orifices 124, 125 du tournant sont venus en regard des orifices respectivement 6, 7, du fond du puits et les broches du connecteur peuvent être enfoncées au niveau convenable pour la dialyse.

Du fait de la modification de construction du tournant il apparaît d'autre part que dés le début de la rotation à partir de la position d'introduction illustrée à la figure 20, les ergots 29 (figure 3) viennent passer grâce aux arrondis 119, 120 au dessus du plan supérieur de la coupelle 70 en comprimant la rondelle en élastomère 71 assurant donc une parfaite étanchéité à la fermeture des orifices 6, 7 du fond du puits contre la face inférieure en élastomère du tournant composite 15. Et cette pression est maintenue pour toute position angulaire du tournant autre que la position d'introduction initiale montrée à la figure 20. On est ainsi assuré d'une parfaite étanchéité du système dans toutes les positions d'utilisation.

En se référant aux figures 22 et 26, on a illustré la position permettant le changement du tournant grâce à l'utilisation d'un extracteur. Dans la variante ainsi montrée l'outil extracteur 159 est légèrement modifié par rapport à l'outil 59 illustré à la figure 13, essentiellement en ce qu'il comporte à la place des deux trous borgnes 67, 68 de la figure 13 deux pions en relief 167, 168. Egalement la rainure circulaire 63b qui faisait dans le mode de réalisation de la figure 13 un quart de tour court ici sur un angle de 120° entre la rainure rectiligne d'entrée 163a et la rainure rectiligne de sortie 163c, ces deux rainures étant comme dans le mode de réalisation de la figure 13 parallèles à l'axe de l'extracteur.

La modification de fonctionnement va être décrite maintenant.

Pour effectuer le changement d'un tournant, on présente l'extracteur comme il a été décrit précédemment en relation avec la figure 13, la bonne orientation étant assurée par l'introduction du doigt de positionnement 28 dans la rainure d'entrée 163a de l'outil extracteur. Dans cette position de rotation les pions 167, 168 viennent se positionner dans les orifices 124, 125 du tournant 115. Avantageusement, ils ont un diamètre légèrement plus important que celui des orifices 124, 125, assurant ainsi un verrouillage élastique du tournant 115 sur l'outil extracteur lorsque les pions 167, 168 ont été convenablement introduits dans les orifices 124, 125 formés dans la matière élastomère de la rondelle 71.

Cette opération étant faite, il faut tourner sur un angle de 120°, comme illustré à la figure 22, l'ensemble de l'outil extracteur et du tournant pour amener les orifices 72, 73 en regard respectivement des orifices 7 et 6 du fond du puits. Dans cette position il est alors possible d'introduire comme décrit précédemment notamment en relation avec les figures 15 à 18 les broches 65, 66 dans l'extracteur et qui vont venir obturer les orifices 6 et 7 du fond du puits. Les orifices 72, 73 ont avantageusement un diamètre légèrement supérieur à celui des broches, présentant par exemple un diamètre de l'ordre de 2,10 mm si les broches ont un diamètre de 2mm, le même diamètre de 2mm étant prévu pour les orifices 6 et 7 et pour les orifices 124, 125. Le jeu ainsi formé permet l'enlèvement aisé du tournant 115 qui reste verrouillé sur l'extracteur grâce aux pions 167, 168 introduits dans les orifices 124, 125, l'extraction étant autorisée du fait que l'ergot 28 de positionnement est cette fois venu au droit de la rainure rectiligne 163c.

**Revendications**

1. - Dispositif implantable pour accès au système circulatoire sanguin du type comprenant essentiellement un raccord en té (T) dont les extrémités débouchantes de la barre ou branche (2) sont reliées à des veines, artères ou prothèses vasculaires (11, 12) et dont l'extrémité de la hampe formant puits (3) d'accès, peut être connectée à tout système extérieur d'échange, par exemple pour la dialyse du sang d'un patient, et dans lequel la connexion/déconnexion avec l'extérieur est obtenue au moyen d'un organe monté dans ledit puits (3) formant tournant (15) comportant deux orifices (24, 25) qui selon la position en rotation du tournant (15) sont mis en communication ou hors de communication avec le volume intérieur (4) de la branche (2) du té et qui d'autre part peuvent être reliés à l'entrée·et à la sortie dudit système extérieur, caractérisé en ce que ledit tournant (15) se présente sous la forme d'un disque sensiblement cylindrique circulaire dont le diamètre extérieur correspond sensiblement au diamètre intérieur du puits (3) comportant des moyens coopérant avec des moyens complémentaires dudit puits, pour assurer au moins dans la position de déconnexion précitée un appui en pression du disque sur le fond du puits et une étanchéité de la fermeture du puits.

2. - Dispositif selon la revendication 1, caractérisé en ce que lesdits moyens complémentaires consistent en des rampes (19, 20) formées sur le disque (15) coopérant avec des ergots (29) formés sur la paroi intérieure du puits (3).

3. - Dispositif selon la revendication 1 ou la revendication 2 caractérisé en ce que ledit tournant (115) comporte assemblée dans une enveloppe métallique (70) en forme de coupelle, une matière élastomère (71) biocompatible faisant saillie en dehors de la coupelle sur sa face libre venant en regard du fond du puits.

4. - Dispositif selon l'une des revendications précédentes caractérisé en ce que le fond du puits (3) est fermé par une paroi (8) sensiblement plane cylindrique circulaire de faible épaisseur sur laquelle vient porter la face en regard du tournant (15) et comportant deux orifices (6, 7) venant en face et en concordance des orifices (24, 25) formés sur le tournant (15) dans la position de connexion de ce dernier.

5. - Dispositif selon l'une des revendications précédentes, caractérisé en ce que le diamètre des orifices (6, 7) formé dans le fond du puits (3) est sensiblement identique à celui des orifices (24, 25) formés dans le tournant (15).

6. - Dispositif selon l'une des revendications précédentes, caractérisé en ce qu'il comprend un dispositif connecteur (30) pour la liaison au système extérieur comprenant un bouchon (31) pouvant venir s'engager dans le puits (3), lequel bouchon est traversé de façon étanche par deux broches creuses (34, 35) susceptibles de passer à travers les orifices précités (24, 25) formés dans le tournant (15) ; ce connecteur (30) comprend en outre des moyens de guidage et d'orientation dans le puits tels qu'au moins une rainure (43) formée dans sa paroi latérale coopérant avec un doigt (28) prévu sur la paroi du puits (3) imposant un mouvement d'introduction du connecteur (30) comportant au moins un premier déplacement d'enfoncement jusqu'à pénétration des broches (34, 35) dans les orifices (24, 25) du tournant (15) alors en position d'obturation, puis un mouvement de rotation jusqu'à amener le tournant (15) en position d'ouverture, puis un nouvel enfoncement rectiligne jusqu'à pénétration des broches au niveau souhaitable dans le volume intérieur (4) de la branche (2) du té, les mouvements de retrait du connecteur (30) étant inverses de ceux de l'introduction et entraînant simultanément l'obligation de positionner en obturation le tournant (15) avant retrait du connecteur.

7. - Dispositif selon l'une des revendications précédentes, caractérisé en ce qu'il comprend une bague de verrouillage sur la collerette (50) du puits (3) permettant par rotation de la bague sur elle-même le verrouillage en translation du connecteur (30) dans la bague sur le puits (3) lorsque le connecteur est en place.

8. - Dispositif selon la revendication 6 ou la revendication 7, caractérisé en ce qu'il comprend un dispositif extracteur (59) comportant comme le connecteur (30) un bouchon (60) pouvant venir s'engager dans le puits (3), lequel bouchon (60) comporte deux orifices (61, 62) pour le passage de broches (65, 66) susceptibles de passer à travers les orifices précités (24, 25) formés dans le tournant (15) ; cet extracteur (59) comprend en outre des moyens de guidage et d'orientation dans le puits tels qu'au moins une rainure (63) formée sur sa paroi latérale coopérant avec un doigt (28) prévu sur la paroi du puits (3) imposant un mouvement d'introduction de l'extracteur comportant au moins un premier déplacement d'enfoncement jusqu'à pénétration des broches (65, 66) dans les orifices (24, 25) du tournant (15) alors en position d'obturation, puis un mouvement de rotation jusqu'à amener le tournant (15) en position d'ouverture; dans cette position, les broches (65, 66) peuvent être enfoncées à travers les orifices (6, 7) de la paroi (8) du fond du puits qu'elles obturent, puis, les broches (65, 66) restant en place, l'extracteur (59) peut être retiré dans un mouvement d'extraction parallèle à l'axe (17) du puits en entraînant avec lui le tournant (15) grâce à la prévision d'au moins une rainure (63c) parallèle à l'axe du puits et dans laquelle est engagé ledit doigt (28) dans cette position en rotation de l'extracteur.

9. - Dispositif selon la revendication 8 caractérisé en ce que le tournant (15) comporte sur sa face extérieure au moins deux pions (26, 27) ou analogues susceptibles de s'engager à force dans les alésages de trous borgnes (67, 68) coopérant de l'extracteur.

10. - Dispositif selon la revendication 8 caractérisé en ce que le tournant (115) comporte, le traversant, quatre orifices (124, 125, 72, 73) disposés par paires en vis-à-vis diamétral correspondant à la mise en concordance desdits orifices avec ceux (6, 7) du fond du puits pour deux positions en rotation différentes, l'une correspondant à la position de dialyse, l'autre à la position de changement du tournant avec passage correspondant des broches (65, 66).

11. - Dispositif selon l'une des revendications précédentes caractérisé en ce qu'il comprend un bouchon (53) de protection/verrouillage du dispositif venant se bloquer élastiquement et de façon étanche par dessus la collerette (50) du puits.

EP 0 318 358 A1

FIG.1

1

12

11

FIG.2

28

50

51

3

52

1

9

10

2

FIG.3

17

28

15

50

1

5

29

19

3

11

12

18

6

8

7

4

6

8

7

2

2

13

FIG.3

FIG. 4

FIG. 5

FIG. 19

FIG.6

FIG.7

FIG.8

FIG.13

EP 0 318 358 A1

FIG_9

FIG_10

FIG_11

FIG_12

FIG.14

FIG.15

FIG.16

FIG.17

FIG.18

FIG.20

FIG.21

FIG.22

FIG_23

FIG_24

FIG_25

FIG_26

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.3) |
|---|---|---|---|
| X,D | US-A-4 108 173  (SLIVENKO) <br> * colonne 2, ligne 46 - colonne 3, ligne 43; colonne 4, lignes 13-43; revendications 1-3; figures 1-11 * <br> --- | 1,5 | A 61 M   1/00 |
| E | WO-A-8 707 509  (BIOMASYS) <br> * page 4, ligne 13 - page 5, ligne 16; page 10, lignes 1-14; revendications 1-3,5-8,14; figures 1-15 * <br> --- | 1 | |
| A | FR-A-2 387 043  (GENERAL ATOMIC COMPANY) <br> * page 3, lignes 29-40; page 4, lignes 16-40; page 5, ligne 15 - page 6, ligne 19; revendications 1-3; figures 1-14 * <br> --- | 1,7 | |
| A | EP-A-0 078 565  (RENAL SYSTEMS) <br> * page 8, ligne 18 - page 9, ligne 12; revendications 1,6-8; figures 1,7,8,14 * <br> --- | 1,4-6, 11 | |
| A | EP-A-0 087 189  (RENAL SYSTEMS) <br> * page 7, lignes 6-25; page 10, ligne 24 - page 12, ligne 12; page 13, lignes 5-19; figures 9-20 * <br> ----- | 1,6-8 | **DOMAINES TECHNIQUES RECHERCHES (Int. Cl.3)** <br><br> A 61 M   1/00 |

Le présent rapport a été établi pour toutes les revendications

| Lieu de la recherche | Date d'achèvement de la recherche | Examinateur |
|---|---|---|
| BERLIN | 13-02-1989 | MONNE E.M.B. |

EPO FORM 1503 03.82 (P0402)